# EUROPEAN PATENT SPECIFICATION

(11) **EP 1 761 639 B1**
(45) Date of publication and mention of the grant of the patent: **02.01.2013**
(21) Application number: 05707697.8
(22) Date of filing: 03.03.2005
(51) Int. Cl.: C12Q 1/68

(54) **ANALYSIS OF METHYLATED NUCLEIC ACID**
ANALYSE METHYLIERTER NUKLEINSÄURE
ANALYSE D'ACIDE NUCLEIQUE METHYLE

(30) Priority: 18.06.2004 GB 0413688
(43) Date of publication of application: 14.03.2007
(73) Proprietor: Novartis Forschungsstiftung, Zweigniederlassung, 4058 Basel (CH)
(72) Inventor: SCHUEBELER, Dirk, CH-4053 Basel (CH); WEBER, Michaël, Philippe, F-68300 Saint Louis (FR)
(74) Representative: Marshall, Cameron John
(86) International application number: PCT/EP2005/002251
(87) International publication number: WO 2005/123942

(56) References cited:
- WO-A-02/00842
- WO-A-98/56952
- WO-A-02/101353
- WO-A-2004/065625
- PIYATHILAKE C.J. ET AL.,: "immunohistochemical evaluation of global dna methylation: comparison with in vitro radiolabeled mthyl incorporation assay" BIOTECHNIC&HISTOCHEMISTRY, vol. 18, November 2000 (2000-11), pages 251-258, XP009051676

## Description

### TECHNICAL FIELD

The present invention relates generally to methods and materials for use in the enrichment and analysis of methylated DNA and identification of aberrantly methylated sites in disease.

### BACKGROUND ART

Methylated nucleotide bases have been found in both prokaryotes and eukaryotes (Achwal et al., 1983). Those found in eukaryotes include 5-methylcytosine, 6-methyladenine and 7-methylguanine in DNA (Achwal et al., 1983) and 5-methylcytosine (Hernandez-Blazquez et al., 2000) and 7-methylguanosine (Tebib et al., 1997) in RNA. The reversible methylation of cytosines, usually at CpG dinucleotides, is a common DNA modification in higher eukaryotes including plants and animals.

DNA methylation can lead to transcriptional repression and thus is involved in gene regulation and imprinting of mammalian genes such as those for insulin growth factor and its receptor, and the *Xist* gene. DNA methylation is an epigenetic regulator since the modification does not change the DNA sequence but is inherited through cell division. Aberrant DNA methylation can cause disease. In particular, aberrant DNA methylation may result in increased expression of proto-oncogenes or decreased expression of tumour suppressor genes. Thus, misregulation of DNA methylation is a phenotypical hallmark of many human cancers (Jones and Baylin, 2002). The emerging picture is that of a global reduction in the amount of methylated cytosine with coinciding hypermethylation of a subset of promoters, which in some cases are linked to inactive tumour suppressor genes. However, the genomic location of this hypomethylation is unknown, as is the frequency and specificity of aberrant promoter methylation.

Given the relevance of DNA methylation for normal and abnormal cell function and its potential as a drug target and as a diagnostic tool in oncology, technical approaches to identify DNA methylation are highly desirable (Fazzari and Greally, 2004). Using antibodies specific to methylated nucleic acids is mentioned in WO 02/00842, WO 02/101353 and WO 2004/065625.

Current available protocols to detect methylated DNA often require the use of modification-sensitive restriction endonucleases (MSRE) or differential base modification using chemicals e.g. bisulfite, hydrazine or permanganate (Rein et al., 1998) followed by DNA sequence analysis. Although differential base modification methods can map a methylated base to a precise nucleotide position in a stretch of DNA, these methods are too laborious to be applied to large-scale (genome-wide) analysis. The use of methylation sensitive restriction enzymes can be used for genome-wide analysis but the number of sites that can be examined is limited by the number of appropriate restriction sites in the nucleic acid. This means that such methods cannot map the location of a modified base on a chromosome so precisely. Moreover, the current chemical and enzymatical detection methods can only be performed with relatively high quality DNA.

Thus it can be seen that novel methods for enrichment or detection of methylated nucleic acid as well as identification of the DNA aberrantly methylated in disease would provide a contribution to the art.

### DISCLOSURE OF THE INVENTION

The present inventors have demonstrated that antibodies specific for methylated nucleosides can be utilised in methods for efficiently enriching and identifying specific methylated nucleic acids in samples of nucleic acid fragments. Using this novel approach the inventors have observed up to 120 fold enrichment of methylated sequences over an unmethylated control as detected by Real-time-PCR. The methods are independent of the sequence of the nucleic acid fragments, do not require a high quality of nucleic acid, and are readily susceptible to large scale genomic analysis, for instance when combined with conventional DNA sequence detection methods. In addition to permitting the determination of which sequences in a sample are methylated, the inventors have also demonstrated that enrichment by the immunoprecipitation-based methods of the invention is dose dependent and can thus be used to quantify the extent of methylation of a sequence.

Antibodies specific to modified bases have previously been used for detection the overall amount and general location of modified bases. For example, antibodies specific to 5-methylcytidine (m⁵C) have been shown to react with m⁵C in mammalian DNA bound to nitrocellulose paper (Achwal et al., 1983; Achwal & Chandra, 1982). Immunofluorescence has also been used to determine chromosomal regions with a high frequency of m⁵C (Barbin et al., 1994). Mouse monoclonal antibody against 5-methylcytidine has also been used previously to detect alterations in the urinary excretion of nucleosides by cancer patients (Tebib et al., 1997) and to visualize the distribution of methylated sequences along mammalian chromosomes in normal and malignant cells (Hernandez-Blazquez et al., 2000; Mayer et al., 2000). However such antibodies have not previously been taught or suggested for enrichment of methylated nucleic acids in samples of nucleic acid.

Thus, in a first aspect, the present invention provides a method for enriching methylated nucleic acid fragments in a sample of nucleic acid fragments comprising the steps of:
(a) contacting the sample of nucleic acid fragments with an antibody specific to a methylated nucleoside under conditions suitable for binding of the antibody to the methylated nucleoside;
(b) selecting nucleic acid fragments bound to the antibody.

Prior to selecting the nucleic acid fragments bound to the antibody specific to a methylated nucleoside, the methylated and non-methylated fragments may be separated on the basis of binding of the methylated fragments to the antibody.

The invention further comprises a step of separating the strands of any double-stranded nucleic acid fragments in the sample to form a sample of single-stranded nucleic acid fragments, before contacting the sample of single-stranded nucleic acid fragments with an antibody specific to a methylated nucleoside.

In preferred embodiments the invention provides a method for characterising or identifying methylated nucleic acid fragments from a sample of nucleic acid fragments, the method further including the step of:
(c) characterising one or more of the methylated nucleic acid fragments.

By "enrichment" is meant an increase in the proportion of a particular category of nucleic acid fragment in or from a sample of nucleic acid fragments. Preferably the enrichment is at least 5, 10, 20, 30, 50, or 100 fold.

In another aspect the invention provides the distribution of DNA methylation in disease and thereby targets for therapeutic intervention as well as diagnostics, prognostics and surrogate markers useful in the fight against cancer and other diseases.

Some preferred embodiments will now be discussed.

### Nature of the nucleic acid

Although the above described method may be applied to a sample of any type of nucleic acid, preferably the nucleic acid is DNA.

Examples of methylated nucleosides include methylated cytidine (e.g. 5-methyl cytidine), methylated adenosine (e.g. 6-methyl adenosine) and methylated guanosine (7-methyl guanosine). In a preferred embodiment, the methylated nucleoside is methyl cytidine. More particularly preferred, the methylated nucleoside is 5-methyl cytidine.

### Nature of the sample

The sample may be any which it is desired to analyse.

The low requirement on the quality of the nucleic acid to be tested makes this protocol suitable for the study of formalin fixed specimens, whose nucleic acid is partly degraded by the formalin. As discussed below, the method may be used to relate changes in nucleic acid methylation to clinical history.

The skilled person can readily determine how to fragment nucleic acid to produce a sample of nucleic acid fragments. For example, genomic DNA may be fragmented using shearing (e.g. by sonication) or digestion with restriction enzymes such as *Alu*I.

Once obtained, the sample of nucleic acid fragments is preferably suspended in a liquid (e.g. a buffer suitable for antibody binding).

### Denaturation of nucleic acid

The specificity of antibodies directed against nucleotide modifications is higher if the nucleic acid is single stranded. Thus, the efficiency of enrichment and detection of methylated nucleotides is greatly increased if the nucleic acid molecules in the sample are single stranded. Therefore, it is desirable to separate the strands of any double-stranded nucleic acid molecule present in the sample, as specified in claim 1.

Denaturation (separating the strands of a double-stranded molecule) is most readily done by heating the nucleic acid. The skilled person can readily determine a temperature and length of heating time suitable for denaturing the nucleic acid that they are interested in. Heating to 95°C for 10 minutes has been found to be effective for DNA for use in the present invention.

### Nature of the antibody

Antibodies specific to many methylated bases are available commercially. For example a mouse monoclonal antibody against m⁵C is available from Eurogentec S.A. (Belgium) and a rabbit polyclonal serum is available from Megabase Research Products (USA). Polyclonal rabbit antisera against other methylated bases (6-methyladenosine and 7-methylguanosine) are available (Megabase Research Products, USA).

Alternatively antibodies specific to methylated bases can be made using conventional techniques (see e.g. Roitt et al. in "Immunology 5th edition" - Pub. 1997 by Moseby International Ltd, London).

The term "antibody" as used herein should be construed as covering any specific binding substance having a binding domain with the required specificity. Thus, this term covers antibody fragments, derivatives, functional equivalents and homologues of antibodies, including any polypeptide comprising an immunoglobulin binding domain, whether natural or synthetic. Chimaeric molecules comprising an immunoglobulin binding domain, or equivalent, fused to another polypeptide are therefore included. Cloning and expression of chimaeric antibodies are described in EP-A-0120694 and EP-A-0125023.

For example, it has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341, 544-546, 1989) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., Science, 242, 423-426, 1988; Huston et al., PNAS USA, 85, 5879-5883, 1988); (viii) bispecific single chain Fv dimers (PCT/US92/09965) and (ix) "diabodies", multivalent or multispecific fragments constructed by gene fusion (WO94/13804; Holliger et al., Proc. Natl. Acad. Sci. USA 90 6444-6448, 1993).

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen binding site: antigen binding sites are formed by the association of the first domain of one polypeptide within the multimer with the second domain of another polypeptide within the multimer (WO94/13804).

Preferably the antibody is specific for methylcytidine. More preferably, 5-methylcytidine.

### Conditions suitable for binding of the antibody specific to the methylated nucleoside

The skilled person can readily determine the conditions suitable for binding of the first antibody to the methylated nucleoside in a liquid phase. In particular, it is important to maintain an appropriate ionic balance in the sample so that the antibody can bind effectively to the methylated nucleoside. For example, the pH of the sample can be controlled by addition of suitable buffers such as sodium phosphate, which will maintain the pH at approximately 7.0. Salts, such as sodium chloride may also be added to the buffer and/or the sample.

Maintenance of the sample at approximately 1 to 5°C whilst contacting it with the nucleic acid may be preferred.

Binding of methylated nucleic acid to the first antibody 'tags' the methylated nucleic acid. This 'tagging' allows methylated nucleic acid to be separated from non-methylated nucleic acid.

### Nature of the separation step

Preferably, prior to the selection step, methylated and non-methylated nucleic acid fragments are separated on the basis of binding of the first antibody to the methylated nucleoside. This may be done by any method known to those skilled in the art.

In preferred embodiments, the separation is performed by attaching or binding the antibodies to a solid phase or substrate (the terms are used interchangeably) and separating this solid phase from the sample liquid phase. Thus addition of a solid substrate that binds specifically to the first antibody facilitates the separation of methylated nucleic acid from non-methylated nucleic acid. Specific binding of the solid substrate to the first antibody can be achieved by using a solid substrate that comprises a second antibody specific for the first antibody. For example, if the first antibody (i.e. the antibody specific to a methylated nucleoside) is a mouse anti-m⁵C antibody, a goat anti-mouse antibody would be suitable.

A solid substrate in the form of beads is particularly useful as this gives a large surface area over which binding can occur. Magnetic beads such as Dynabeads (Dynal Biotech) allow simple separation of methylated and non-methylated nucleic acid as the beads (and therefore the nucleic acid bound to them) can be easily removed from a sample using a magnet. Alternatively, the solid substrate could be separated from the non-bound nucleic acid using techniques such as centrifugation and/or filtration. The skilled person can readily determine a suitable way to separate the solid substrate he is using from non-bound (i.e. non-methylated) nucleic acid.

### Characterising the methylated nucleic acid

Prior to characterising the methylated nucleic acid fragments, it is desirable to detach the methylated nucleic acid from the first antibody (and the solid substrate if used). The skilled person can readily determine such detaching methods in which nucleic acid is not damaged during the detaching process.

For example, a nucleic acid fragment may be detached from an antibody by digesting the antibody. This may be achieved by incubating the nucleic acid fragments bound to the first antibody with a proteinase such as Proteinase K.

Slightly altering the pH around the nucleic acid bound to the first antibody may weaken the binding between the antibody and methylated nucleic acid, further facilitating detachment. This may be achieved by adding a suitable buffer (e.g. 50mM Tris pH 8.0) to the methylated nucleic acid and antibody bound to it.
The skilled person can readily determine other suitable ways to do this. EDTA (Ethylenediaminetetraacetic acid) and SDS (sodium dodecyl sulphate) may also be added to the buffer.

Once it has been detached from the first antibody and the solid substrate, the methylated nucleic acid can be analysed further - for example to determine the amount present, all or part of the sequence of the methylated fragment and\or the sequence or position of the methylation site.

This step may be preceded by further treatment of the nucleic acid. For example where the methylated nucleic acid is DNA it may be extracted (e.g. in phenol and chloroform) and subsequently precipitated (e.g. with ethanol).

Conventional nucleic acid analysis techniques are then applied to the methylated nucleic acid. For example, the presence of sequences of interest in the methylated nucleic acid may be determined using techniques such as PCR, slot blots, microarrays etc. such as are well known to those skilled in the art.

For example analysis may employ a microchip system comprising a microarray of oligonucleotides or longer DNA sequences on a glass support. Sample nucleic acid (e.g. fluorescently labelled) may be hybridised to the oligonucleotide array and sequence specific hybridisation may be detected by scanning confocal microscopy and analysed automatically (see Marshall & Hodgson, Nature Biotechnology 16: 27-31, 1998; also Nature Cell Biology August 2001 volume 3 issue 8 pp E190 - E195 "Navigating gene expression using microarrays - a technology review" Almut Schulze and Julian Downward). A list of currently used techniques in microarray assembly and DNA detection can be found in the book "DNA Microarrays: A Molecular Cloning Manual", eds. Bowtell and Sambrook, CSHL 2002.

Some other aspects of the invention will now be discussed.

### Genome analysis

Since the nucleic acid fragments isolated using the methods described above can be analysed by either standard PCR or slot blot hybridisation this method can be applied to large-scale (genome-wide) analysis using microarrays.

Thus the invention provides a method of characterising the methylation status of a DNA sample (for example from an organism genome) comprising:
(i) fragmenting the genome
(ii) performing a method of the invention as described above.

By "methylation status" is meant whether, and/or to what extent, the nucleic acid sequence is methylated. The extent of methylation may be measured as which nucleotides in the sequence are methylated and/or the proportion of nucleotides in the sequence which are methylated.

### Methods of comparing methylation of nucleic acids

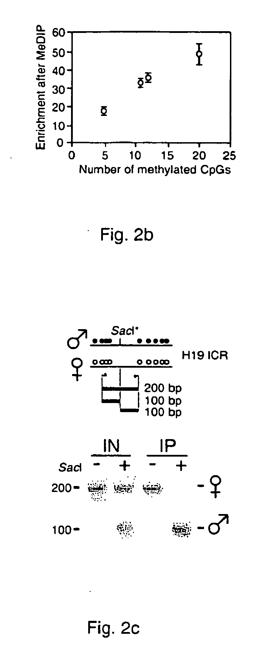

As discussed above, in the methods of the present invention the enrichment is dose dependent (i.e. the enrichment is proportional to the number of methylated nucleosides) (see Example 2 and Figure 2B). This means that not only can the invention be used to determine if a gene is methylated, it can be used to quantify or compare the extent of methylation of that gene.

Thus the invention provides a method of comparing the methylation of different, corresponding, nucleic acids or nucleic acid samples, comprising:
(i) performing a method of the invention as described above on each nucleic acid or sample,
(ii) comparing the results at step (d).

The methods may be performed separately, or multiplexed (provided the nucleic acids or samples are distinguishable). Typically nucleic acids may be distinguished by sequence markers or labels.

In one embodiment the invention may be used for detecting differentially methylated alleles in a sample - for example of imprinted genes.

"Imprinted genes" are genes whose alleles have different expressivity or penetrance depending on whether they are inherited from the male or the female parent. Imprinting can be both developmental-stage specific or tissue specific. If the maternal and paternal alleles of a gene are differentially methylated, they will be enriched to differing extents in a sample of nucleic acid subjected to the methods of the present invention.

An example of an imprinted gene whose alleles are differentially methylated is the *H19* ICR in mice (see Example 3 and Figure 2C). This locus contains a CpG island. CpG islands are stretches of sequences around lkb long, often occurring at the 5' ends of genes, that contain a high density of CpG dinucleotides. This CpG island is not methylated in the maternal allele, but methylated in the paternal allele (see Example 3 and Figure 2C). When applied to a sample of fragments of the mouse genomic DNA, the methods of the present invention will enrich the paternal allele but not the maternal allele. The skilled person can readily determine a suitable technique for determining whether the maternal or paternal allele that has been enriched in the sample. The use of a 'marker' for either the maternal or the paternal allele is particularly useful. For example, the *H19* ICR allele from *Mus* spretus contains a polymorphic *Sac*I restriction site that is not present in the *Mus musculus domesticus H19* ICR allele. Thus, a *domesticus* x *spretus* hybrid will have one allele with the *Sac*I restriction site and one without. PCR amplification using primers for the *H19* ICR followed by treatment of the PCR product with *Sac*I results in a single 200bp fragment for the *domesticus* allele and two 100bp fragments for the *spretus* allele. The size of the fragments obtained from an 'enriched' sample therefore shows whether the maternal, paternal or both alleles have been enriched.

### Diagnosis or prognosis

As discussed above, aberrant DNA methylation can cause disease. In particular, aberrant DNA methylation may result in increased expression of proto-oncogenes or decreased expression of tumour suppressor genes and is associated with many human carcinomas.

The methods of the invention may be used to screen and identify aberrant nucleic acid methylation sites associated with disease states, or for diagnosis or prognosis of disease or disease progression e.g. in cancer.

Novel aberrant nucleic acid methylation sites associated with disease states may be identified by performing the method of the invention on nucleic acid samples from diseased and non-diseased individuals and comparing the results.

The invention further provides a method of diagnosis in an individual of a disease associated with methylation of a specific nucleic acid sequence, comprising:
(i) performing a method of the invention as described above on a nucleic acid sample from the individual to characterise whether the specific nucleic acid sequence is methylated, and,
(ii) correlating the result with the disease state of the individual.

The invention further provides for the detection of changes in nucleic acid methylation over time (e.g. to relate this to clinical history, and hence the diagnosis or prognosis of a disease associated with alterations in methylation of a nucleic acid sequence).

Such a method may include the steps of:
(i) Obtaining a sample of nucleic acid fragments from a patient at least two time points.
(ii) Carrying out the method of the invention on each sample of nucleic acid fragments for each time point to characterise whether, and/or to what extent, the nucleic acid sequence is methylated.

Preferably the sample of nucleic acid fragments is obtained from a patient using the following protocol:
(a) obtaining a tissue specimen from the patient;
(b) extracting nucleic acid from each tissue specimen to provide a sample of nucleic acid;
(c) fragmenting the sample of nucleic acid to give a sample of nucleic acid fragments;

The method for detection of changes in nucleic acid methylation over time may also further comprise
(iii) recording the clinical symptoms of a disease observed in the patient at each time point, and
(iv) comparing the clinical symptoms recorded at each time point with the methylation status of the nucleic acid sequence of interest at each time point.

The method for detection of changes in nucleic acid methylation may be carried out in any appropriate order. For example, the extraction and analysis steps may be carried out at or shortly after each time point. Alternatively, the specimens or samples may be stored and extraction of nucleic acid and\or comparison of the recorded clinical symptoms with methylation status carried out for a plurality of samples together. For example tissue specimens may be frozen or fixed in formalin for storage.

Preferably the disease associated with methylation of a specific nucleic acid sequence or alterations in methylation of a nucleic acid sequence is cancer.

The invention will now be further described with reference to the following non-limiting Figures and Examples.

### Screening for modulators and methods of treatment

Once a target gene has been identified it may be used for drug screening purposes to seek to identify potential anti-cancer drug. Such screens may involve the expression of the protein encoded by the target gene and chemical agents contacted with the protein in order to ascertain whether or not the chemical agent has any effect on the proteins activity.

Assays for protein activities of known function are know in the art. Generally such assays are termed functional assays and may be conducted *in vitro* in a cell free or cell based system. Where a functional assay is available, it is to be preferred to a ligand binding assay.

Assays for proteins of unknown function typically rely on assessment of ligand binding only, but other assays based on disturbance of chemical levels are well known to those of skill in the art.

The provision of candidate chemicals for use in the present invention are well known to those skilled in the art. For example libraries of compounds can be easily synthesised and tested. This is well described for example in: Applications of combinatorial technologies to drug discovery, 2. Combinatorial organic synthesis, library screening techniques, and future direction, J. Med. Chem., 1994, 37, 1385-1401.

For proteins of unknown function, the ligand binding assays outlined herein will also define a group of candidate chemicals. However, this group is likely to be large, since binding may occur to a number of different sites on the exposed surface of the protein, and binding alone does hot predict the effect of ligand binding on the activity of the protein. Stringent selection among the candidate chemicals for those with the greatest affinity will define a set of chemicals small enough to be tested.

An alternative or additional procedure is to express the protein target in a cell which has been manipulated genetically to contain a sensor for calcium ions, cyclic AMP or other components of cell signalling pathways. For example, permanent cell lines of any suitably origin may be transfected, and lines expressing the protein permanently selected. In many cases, expression of an unknown protein will cause a shift in the level of cell signalling components, which will be detected by the sensor and can be read, for example, as a fluorescent or luminescent signal. The difference between the protein-expressing cells and control cells forms the basis of the assay. Effects of chemicals on the difference between protein expressing and control lines are assessed.

Nucleic acid encoding polypeptides according to the invention may also be used in gene therapy. In gene therapy applications, genes are introduced into cells in order to achieve in vivo synthesis of a therapeutically effective genetic product, for example for replacement of a defective gene. Gene therapy includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes in viva. It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA, 83:4143-4146,1986). The oligonucleotides can be modified to enhance their uptake, e.g., by substituting their negatively charged phosphodiester groups by uncharged groups.

There are a variety of techniques available for introducing nucleic acids into viable cells. The techniques vary depending upon whether the nucleic acid is transferred into cultured cells in vitro, or in vivo in the cells of 1 the intended host. Techniques suitable for the transfer of nucleic acid into mammalian cells in vitro include the use of liposomes, electroporation, microinjection, cell fusion, DEAE-dextran, the calcium phosphate precipitation method, etc. The currently preferred in vivo gene transfer techniques include transfection with viral (typically retroviral) vectors and viral coat protein-liposome mediated transfection (Dzau et al., Trends in Biotechnology, 11: 205-20, 1993). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g., capsid proteins or fragments thereof tropic for a particular cell type, antibodies for proteins which undergo internalization in cycling, proteins that target intracellular localization and enhance intracellular half-life. The technique of receptor mediated endocytosis is described, for example, by Wu et al., J. Biol. Chem., 262: 4429-4432, 1987; Wagner et al., Proc. Natl. Acad. Sci. USA, 87: 3410-3414, 1990). For review of gene marking and gene therapy protocols see Anderson et al., Science, 256: 808-813, 1992.

### DESCRIPTION OF FIGURES

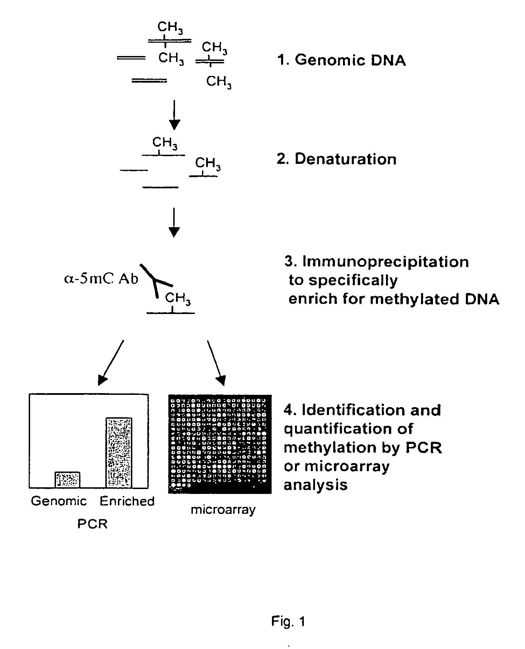

Figure 1: Schematic diagram of a preferred embodiment of the invention and subsequent characterising step. Genomic DNA of desired average length (either generated by digestion with restriction enzymes or by shearing) is denatured and methylated DNA is isolated by incubation with an antibody directed against 5-methylcytidine. The antibody for 5-methylcytidine binds to methylated sequences. These are separated from non-methylated sequences and can then be characterised by standard DNA detection methods such as PCR or microarray.

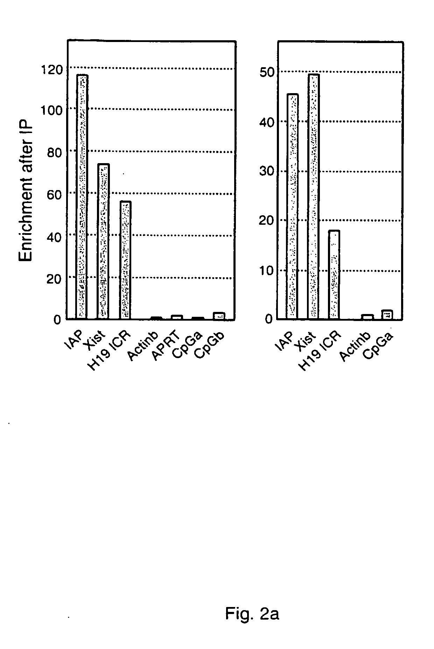

Figure 2A: Detection of methylated sequences in the mouse genome. Enrichment of methylated sequences (IAP Long Terminal Repeat, *Xist* promoter, *X19* ICR) relative to unmethylated CpG islands (*Actinb* and APRT promoters) and control sequences containing no CpG (CpGa and CpGb). The values were calculated by real-time PCR on DNA from *Alu*I digested female gDNA (left) and male sonicated gDNA (right) subjected to the methods of the present invention. IAP = Intracisternal A-Particle. ICR = Imprinting Control Region. APRT = Adenine phosphoribosyltransferase.
Figure 2B: Positive correlation between the enrichment and the number of methylated cytosines on 4 *Alu*I restriction fragments in the *H19* ICR sequence.
Figure 2C: Specific detection of the methylated paternal allele of the imprinted *H19* ICR. The assay was performed on hybrid mouse gDNA (*Mus musculus domesticus* x *spretus),* and parental alleles of the ICR were identified by PCR using a polymorphic *Sac*I restriction site specific for the *spretus* allele. Filled and open lollipops represent methylated and unmethylated CpGs respectively. ICR = Imprinting Control Region. IP = 'enriched' samples. IN = non-'enriched' samples.

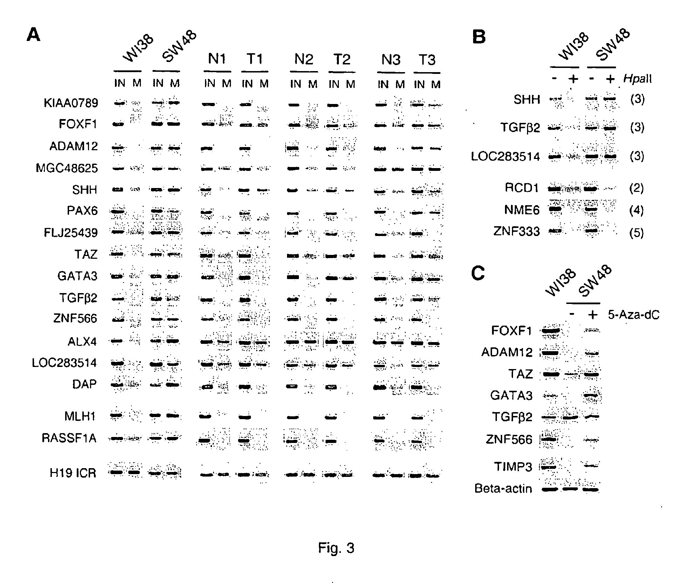

Figure 3: Validation of novel targets for aberrant methylation in colon cancer. A. Primers for PCR were designed for positive clones identified in the microarray analysis as hypermethylated in SW48 cells. DNA methylation was controlled by single-gene PCR on enriched methylated samples prepared from WI38 primary fibroblasts and SW48 colon cancer cells according to the method described above. IN = input genomic DNA, M = enriched methylated DNA. As a separate control the inventors analyzed samples prepared from matched normal colon (N) and colon tumor (T) from three patients. MLH1 and RASSF1A have previously been described to be aberrantly methylated in SW48 cells and in some colon tumors. The imprinted H19 ICR sequence serves as a positive control for methylation. B. Control of methylation status by methylation-sensitive restriction. Genomic DNA was either digested with the methylation-sensitive *Hpa*II enzyme or undigested and used as a PCR template with primers spanning a *Hpa*II-containing PCR fragments in three randomly selected positive clones (top) and negative clones (bottom). The number of *Hpa*II sites within the PCR amplicon is indicated in parenthesis. Presence of a PCR product after *Hpa*II digest reflects DNA methylation in the sample and in each case confirms the above described enrichment analysis. C. Reactivation of silenced genes in SW48 cells by 5-Aza-dC treatment. RT-PCR was performed on cDNA preparation from WI38 fibroblasts and SW48 cells treated (+) or not (-) with 5 µm 5-Aza-dC for 4 days. The beta-acting gene was used as a control.

### EXAMPLES

### Example 1

### Sample preparation

Male mouse *(Mus musculus domesticus)* genomic DNA was fragmented by sonication using a Branson digital sonifier and female mouse *(Mus musculus domesticus)* genomic DNA was fragmented by digestion with *Alu*I (NEW ENGLAND BIOLABS, USA) using conditions recommended by the manufacturer.

### Enrichment

4 *µ*g *Alu*I-digested or sonicated mouse genomic DNA was diluted in 450µL TE (10 mM Tris-HCl pH 7.5, 1mM EDTA) to make a DNA suspension and heated to 95°C for 10 minutes to denature the DNA.

51µL of 10x IP buffer (100mM Na-Phosphate pH7.0, 1.4M NaCl, 0.5% Triton X-100) and 10µL of mouse monoclonal antibody against 5-methylcytidine (EUROGENTEC, #MMS-900P-B) were added to the suspension. The suspension was then incubated for two hours at 4°C with overhead shaking.

30pL of Dynabeads M-280 Sheep anti-mouse IgG (DYNAL BIOTECH, #112.01) were pre-washed in PBS-BSA 0.1% (phosphate buffered saline-bovine serum albumin) and added to the suspension. The suspension was incubated for a further 2 hours at 4°C with overhead shaking.

The beads were washed three times with 700µL of 1x IP buffer (10mM Na-Phosphate pH7.0, 0.14M NaCl, 0.05% Triton X-100) and resuspended in 250µL digestion buffer (50mM Tris pH 8.0, 10mM EDTA, 0.5% SDS).

7µL of proteinase K (10mg/ml) was added to the bead suspension and the bead suspension was incubated at 50°C for 3 hours with horizontal shaking.

DNA was extracted from the bead suspension by organic extraction followed by ethanol precipitation. This was done as follows:
1. Extraction with 1 volume of Phenol.
2. Extraction with 1 volume of chloroform.
3. The remaining aqueous phase was adjusted to 300 mM NaCl and 2 volumes of Ethanol added.
4. Precipitation was performed by centrifugation at 14.000 rpm at room temperature.
5. The resulting DNA pellet was washed with 200 *µ*l of 70 % Ethanol and subsequently dissolved in 50 *µ*L TE.

### Detection of abundance of methylated sequences

The abundance of methylated sequences (IAP Long Terminal Repeat, *Xist* promoter, *H19* ICR), relative to unmethylated CpG islands (Actinb and APRT promoters) and control sequences containing no CpG (CpGa and CpGb) was analysed using real-time PCR.

Results for female mouse *Alu*I digested genomic DNA (left) or sonicated male mouse genomic DNA (right) are given in Figure 2A. Using this novel approach up to 120 fold enrichment of methylated sequences over an unmethylated control was observed.

### Example 2

Mouse *(Mus musculus domesticus)* genomic DNA was fragmented by digestion with *Alu*I. 4 *µ*g of the fragmented DNA was subjected to enrichment as described in Example 1.

The abundance of 4 restriction fragments in the *H19* ICR containing various amounts of methylated CpGs was calculated by real time PCR relative to a negative control containing no CpG.

The result in Figure 2B shows a positive linear correlation between the enrichment and the amount of methylated CpGs in the restriction fragment.

### Example 3

Hybrid mouse (*Mus musculus domesticus* x *Mus spretus*) genomic DNA was fragmented by sonication.

4 *µ*g of the fragmented DNA was diluted in 450µL TE (10 mM Tris-Hcl pH 7.5, 1mM EDTA) to make a DNA suspension and heated to 95°C for 10 minutes to denature the DNA.

The suspension was split into two samples (IN and IP). Sample IP was subjected to enrichment as described in Example 1. Sample IN was not subjected to enrichment.

### Detection of maternal and paternal alleles

Both IN and IP samples were amplified by PCR using primers for the *H19* ICR allele which result in a 200 bp PCR product. The *H19* ICR allele from *Mus spretus* contains a polymorphic *Sac*I restriction site that is not present in the *Mus musculus domesticus H19* ICR allele. Treatment of the PCR product from the *domesticus* allele with *Sac*I leaves the 200bp fragment uncut, whereas treatment of the PCR product from the *spretus* allele with *Sac*I gives two 100bp fragments.

IN and IP PCR products were each split into two sub-samples. The four sub-samples are denoted IN-, IN+, IP-, IP+. IN+ and IP+ were treated with *Sac*I after PCR. IN- and IP- were not treated with *Sac*I.

Figure 2C is a picture of an ethidium-bromide agarose gel showing the DNA fragments found in each sub-sample. Before enrichment, both the paternal *(spretus)* allele and maternal *(domesticus)* alleles are present in the sample. After enrichment, only the paternal allele is present. This demonstrates that only the paternal *(spretus)* allele is enriched by this method. Only the paternal *H19* ICR allele is methylated and so the enrichment method used is specific for methylated DNA.

### Example 4

### Identification of novel targets of aberrant methylation in colon cancer.

Colon cancer cell line SW48 (ATTC, Rockville, MD) was enriched for methylated DNA as described above an the sample the resulting samples subjected to hybridization to a micorarray representing approximately 12000 CpG island probes (UHN Microarray Centre, Ontario) as described the manufacturer, in comparison with human lung fibroblast HFL-1 (ATTC, Rockville, MD) and WI-38 (ATTC, Rockville, MD). For CpG island array hybridization 2 µg of sonicated input DNA was labeled with Cy5-dCTP and the product of one enrichement assay with Cy3-dCTP. Labeling was performed by random priming using the Bioprime labeling kit (Invitrogen), 120 µM of each dATP, dGTP, dTTP, 60 µM dCTP and 60 µM Cy5-dCTP or Cy3-dCTP. By this method the inventors identified regions of DNA hypermethylated exclusively in colon cancer cells. In this group of 193 clones, 108 could be identified unambiguously based on sequence annotation. Of these, only 31 correspond to unique sequences, while 77 represent ribosomal DNA. Such hypermethylation of ribosomal DNA has previously been reported in relation to aging and neoplesia, yet its physiological role remains unclear. Of the unique sequences, 3 clones represent intergenic CpG islands, while the remaining 28 map to 26 different genes. With the exception of two genes, all are located in the promoter region.

To validate the microarray results by single-gene PCR on methylated DNA we designed primers specific for 22 of these candidate genes. These controls confirmed SW48-specific methylation in 70% of all genes. Methylation sensitive restriction was used as a separate independent approach and verified differential methylation on three randomly selected genes. Finally, using RT-PCR, the inventors demonstrated for a subset of these genes transcriptional downregulation in SW48 cells and derepression by treatment with the demethylating agent 5-Aza-dC. This reactivation suggests that the detected methylation is directly repressing the activity of the linked gene. Thus, the method of the present invention with hybridization on a CpG island microarray allows the identification of epigenetically silenced genes in cancer cells.

### 5-Aza-dC treatment and RT-PCR

SW48 cells (1 × 10⁶) were seeded in culture medium and maintained for 24 h before treatment. Cells were then treated with 5µM 5-Aza-dC (Sigma) for 4 days. The medium containing 5-Aza-dC was exchanged 24 h after the beginning of the treatment. Control cells were handled the same way without the addition of 5-Aza-dC. Total RNA was prepared using the RNeasy Mini Kit (Quiagen) and cDNA synthesis was performed on 2 µg of total RNA using the Superscipt first-strand synthesis system (Invitrogen) and oligo-dT primers. PCR reactions were performed on 1/20 of the cDNA preparation. Controls without the RT enzyme were all negative.

*Primers for PCR on MeDIP samples (s= sense; as= antisense)*

| | |
|---|---|
| KIAA0789 s | ATTCAAGGCGCACACTATCCC |
| KIAA0789 as | TGCTGCAGTGGCTTTAAGGAA |
| FOXF1 s | TGCATTTCGGAAGCCACTGT |
| FOXF1 as | AAGAGGCTGAAGCGAAGGAAG |
| ADAM12 s | TGGATCCATTTCACAGGCCT |
| ADAM12 as | AAAAGTTTCCCCCCGTGTGT |
| MGC48625 s | CCTTTCCCATCTTAAGCTCCG |
| MGC48625 as | GCGTCCCAGCGACTTTTTT |
| SHH s | TACCTTTGAGGCCACAGAGCC |
| SHH as | GCGGTTGGTTCTTAAGCCCTA |
| PAX6 s | AACAATTCGGCGCTTTTCGT |
| PAX6 as | TTCTTAAATTCTCCCCGGCC |
| FLJ25439 s | GCTTACAGACTTGCCGCAGAA |
| FLJ25439 as | TCTGATCTCTCAAACTCCCGGA |
| TAZ s | CAAGCCCCGAGTGCAGTTATT |
| TAZ as | ATAATTGCCCGCCTGGAGA |

### HpaII digests

2 µg of genomic DNA was either digested with *Xba*I and *Hpa*II or with XbaI alone. PCR reactions were performed on 25 ng of digested DNA using primers spanning over fragments that contain several *Hpa*II restriction sites. Primers sequences and PCR conditions are available upon request.

### Novel targets of aberrant methylation in colon cancer:

Among the target genes identified using the CpG island array, only the homeobox gene PAX6 -accession no. NM_000280- had already been reported to be methylated in SW48 cells. The GATA3 gene, while not studied previously in colon cancer, was reported to be aberrantly methylated in breast cancer cells. The remaining genes represent novel targets for aberrant hypermethylation in cancer.

These genes are involved in a wide variety of biological functions that include regulation of transcription (FOXF1-accession no. NM_001451-, PAX6, TAZ-accession no. NM_015472-, GATA3-accession no. NM_001002295-), cell cycle progression (TGFB2-accession no. NM_003238-), cell-matrix interactions (ADAM12-accession no. NM_003474-) and apoptosis (DAP-accession no. NM_004394-). Some of them RASL11A -accession no. NM_206827-, FOXF1, TGFB2 and SHH-accession no. NM_000193-.

To assess the relevance of our findings for cancer biology, we determined the methylation status for this set of genes in primary adenocarcinoma and matched normal colon tissue from three patients (Figure 3A). Only one of the genes (AXL4) is methylated to some degree in all three normal colon tissues, suggesting a possible colon-specific methylation. For all remaining genes, methylation in SW48 cells does not reflect tissue specific methylation as they are unmethylated in normal colon or methylated in only one (MGC48625-accession no. NM_182609-) or two (LOC283514-accession no. NM_198849-)of the samples. Interestingly, methylation for these latter genes is not uniform in normal colon. Such differential accumulation of methylation with age has been observed for other genes and might predispose to cancer formation. In summary more than half of the tested genes identified as methylated in SW48 cell line are also methylated in at least one tumour, demonstrating that we identified novel targets for aberrant hypermethylation *in vivo.*

### DOCUMENTS CITED

1. Hernandez-Blazquez, F. J., Habib, M., Dumollard, J. M., Barthelemy, C., Benchaib, M., de Capoa, A., and Niveleau, A. (2000). Evaluation of global DNA hypomethylation in human colon cancer tissues by immunohistochemistry and image analysis. Gut 47, 689-693.
2. Mayer, W., Niveleau, A., Walter, J., Fundele, R., and Haaf, T. (2000). Demethylation of the zygotic paternal genome. Nature 403, 501-502.
3. Tebib, J. G., Reynaud, C., Cedoz, J. P., Letroublon, M. C., and Niveleau, A. (1997). Relationship between urinary excretion of modified nucleosides and rheumatoid arthritis process. Br J Rheumatol 36, 990-995.
4. Achwal, C. W., Iyer, C. A. and Chandra, H. S. (1983) FEBS Lett. 158, 353-358
5. Achwal, C. W. and Chandra, H. S. (1982) FEBS Lett. 150, 469-472
6. Barbin, A., Montpellier, C., Kokalj, V. N., Gibaud, A., Niveleau, A., Malfoy, B., Dutrillaux, B. and Bourgeois, C. A. (1994) Hum. Genet. 94, 684-692
7. Rein, T., DePamphilis, M. L. and Zorbas, H. (1998). Nucleic Acids Research. 26, 2255-2264.
8. Jones, P. A. and Baylin, S. B. (2002). Nat Rev Genet 3, 415-428.
9. Fazzari, M. J. and Greally, J. M. (2004). Nat Rev Genet 5, 446-455.
10. Bowtell and Sambrook (eds.) (2002). DNA Microarrays: A Molecular Cloning Manual. CSHL.
11. Roitt et al. (1997)in 'Immunology' 5th Edition. Moseby International Ltd., London
12. Hollinger, P. et al. (1993) Proc. Natl. Acad. Sci. USA. 90: 644-648.
13. WO94/13804 (CAMBRIDGE ANTIBODY TECH and HOLLIGER, K. P.). Published: 1994-06-23.
14. Marshall& Hodgson (1998) Nature Biotechnology 16: 27-31.
15. Schulze, A. and Downward, J. (2001). Nature Cell Biology August 2001, volume 3, issue 8, pp E190 - E195 "Navigating gene expression using microarrays - a technology review".

## Claims

1. A method for enriching methylated nucleic acid fragments in a sample of nucleic acid fragments comprising the steps of:
(a) contacting the sample of nucleic acid fragments with an antibody specific to methylated nucleoside under conditions suitable for binding of the antibody to the methylated nucleoside;
(b) selecting nucleic acid fragments bound to the antibody specific to the methylated nucleoside,
**characterised in that**:
before step (a): the strands of double-stranded nucleic acid fragments in the sample are separated.

2. The method of claim 1, further comprising the further step of (c) characterising one or more of the methylated nucleic acid fragments.

3. The use of the method of any one of the preceding claims for detecting differentially methylated alleles in a sample of nucleic acid fragments.

4. The method of any one of the preceding claims wherein the proportion of methylated nucleic acid fragments in or taken from the sample of nucleic acid is increased at least 5-fold, at least 10-fold, at least 20-fold, at lease 30-fold, at least 50-fold or at least 100-fold between steps (a) and (b).

5. The method of any one of the preceding claims wherein the nucleic acid fragments are DNA fragments.

6. The method of any one of the preceding claims wherein the methylated nucleoside is methylcytidine.

7. The method of any one of the preceding claims wherein the methylated nucleoside is 5-methylcytidine.

8. The method of any one of the preceding claims wherein selecting nucleic acid fragments bound to the antibody is performed by attaching or binding the antibodies to a solid substrate and separating this solid substrate from the sample liquid phase.

9. The method of claim 8 wherein the solid substrate binds specifically to the antibody specific to a methylated nucleoside.

10. The method of claim 9 wherein the solid substrate comprises a second antibody specific for the antibody specific to the methylated nucleoside.

11. The method of any one of claims 8 to 10 wherein the solid substrate is in the form of beads.

12. The method of any one of claims 8 to 11 wherein the solid substrate is magnetic.

13. The method of any one of the preceding claims further comprising detaching the methylated nucleic acid fragments from the antibody specific to the methylated nucleoside.

14. The method of claim 13 wherein detaching the methylated nucleic acid fragments from the antibody specific to the methylated nucleoside comprises incubating the nucleic acid fragments bound to the antibody with a proteinase.

15. A method of characterising the methylation status of a genomic DNA sample comprising:
(i) fragmenting the genomic DNA sample to give a sample of nucleic acid fragments, and
(ii) carrying out the method of any one of claims 2 to 14 on the sample of nucleic acid fragments obtained in (i).

16. A method of comparing the methylation status of at least two samples of nucleic acid fragments comprising:
(i) carrying out the method of any one of claims 2 to 14 on each sample of nucleic acid fragments, and
(ii) comparing the results obtained in (i) from one nucleic acid sample with that of at least one other nucleic acid sample.

17. A method of diagnosis or prognosis of a disease associated with methylation of a specific nucleic acid fragment in an individual comprising:
(i) carrying out the method of any one of claims 2 to 14 on a sample of nucleic acid fragments from the individual;
(ii) correlating the result obtained in (i) with the disease state of the individual.

18. A method for detection of changes in nucleic acid methylation in a patient over time comprising:
(i) taking a tissue specimen obtained from the patient at a time point;
(ii) repeating step (i) for at least one further time point;
(iii)extracting nucleic acid from each tissue specimen to provide a sample of nucleic acid for each time point, and
(iv) carrying out the method of any one of claims 2 to 14 on each nucleic acid sample for each time point to characterise whether, and/or to what extent, the nucleic acid sequence is methylated.

19. A method for correlating changes in nucleic acid methylation with the clinical symptoms of a disease comprising the steps of claim 18 and further comprising:
(a) recording the clinical symptoms of a disease observed in the patient at each time point, and
(b) comparing the clinical symptoms recorded at each time point with the results obtained in step (iv).

20. The method of claim 18 or claim 19 further comprising storage of each tissue specimen and carrying out step (ii) for a plurality of specimens at the same time.

21. The method of any one of claims 17 to claim 20 wherein the disease in cancer.

## Patentansprüche

1. Verfahren zur Anreicherung methylierter Nukleinsäurefragmente in einer Probe von Nukleinsäurefragmenten, das die folgenden Schritte umfasst:
(a) Inkontaktbringen der Probe von Nukleinsäurefragmenten mit einem gegen methyliertes Nukleosid spezifischen Antikörper unter für die Bindung des Antikörpers an das methylierte Nukleosid geeigneten Bedingungen;
(b) Auswählen von an den gegen das methylierte Nukleosid spezifischen Antikörper gebundenen Nukleinsäurefragmenten,
**dadurch gekennzeichnet, dass**:
vor Schritt (a): die Stränge doppelsträngiger Nukleinsäurefragmente in der Probe getrennt werden.

2. Verfahren nach Anspruch 1, bei dem man ferner in einem weiteren Schritt (c) eines oder mehrere der methylierten Nukleinsäurefragmente charakterisiert.

3. Verwendung des Verfahrens nach einem der vorhergehenden Ansprüche zum Nachweisen differentiell methylierter Allele in einer Probe von Nukleinsäurefragmenten.

4. Verfahren nach einem der vorhergehenden Ansprüche, wobei sich der in der Nukleinsäureprobe vorliegende oder dieser entnommene Anteil methylierter Nukleinsäurefragmente um wenigstens das 5-Fache, wenigstens das 10-Fache, wenigstens das 20-Fache, wenigstens das 30-Fache, wenigstens das 50-Fache oder wenigstens das 100-Fache zwischen Schritt (a) und Schritt (b) erhöht.

5. Verfahren nach einem der vorhergehenden Ansprüche, wobei es sich bei den Nukleinsäurefragmenten um DNA-Fragmente handelt.

6. Verfahren nach einem der vorhergehenden Ansprüche, wobei es sich bei dem methylierten Nukleosid um Methylcytidin handelt.

7. Verfahren nach einem der vorhergehenden Ansprüche, wobei es sich bei dem methylierten Nukleosid um 5-Methylcytidin handelt.

8. Verfahren nach einem der vorhergehenden Ansprüche, wobei das Auswählen von an den Antikörper gebundenen Nukleinsäurefragmenten dadurch erfolgt, dass man die Antikörper an ein festes Substrat anhängt oder bindet und dieses feste Substrat von der Probenflüssigkeitsphase trennt.

9. Verfahren nach Anspruch 8, wobei das feste Substrat spezifisch an den gegen ein methyliertes Nukleosid spezifischen Antikörper bindet.

10. Verfahren nach Anspruch 9, wobei das feste Substrat einen zweiten, für den gegen das methylierte Nukleosid spezifischen Antikörper spezifischen Antikörper umfasst.

11. Verfahren nach einem der Ansprüche 8 bis 10, wobei das feste Substrat in Form von Kügelchen vorliegt.

12. Verfahren nach einem der Ansprüche 8 bis 11, wobei das feste Substrat magnetisch ist.

13. Verfahren nach einem der vorhergehenden Ansprüche, bei dem man ferner die methylierten Nukleinsäurefragmente von dem gegen das methylierte Nukleosid spezifischen Antikörper ablöst.

14. Verfahren nach Anspruch 13, wobei man bei dem Ablösen der methylierten Nukleinsäurefragmente von dem gegen das methylierte Nukleosid spezifischen Antikörper die an den Antikörper gebundenen Nukleinsäurefragmente mit einer Proteinase inkubiert.

15. Verfahren zur Charakterisierung des Methylierungsstatus einer genomischen DNA-Probe, wobei man:
(i) die genomische DNA-Probe unter Erhalt einer Probe von Nukleinsäurefragmenten fragmentiert und
(ii) das Verfahren nach einem der Ansprüche 2 bis 14 an der unter (i) erhaltenen Probe von Nukleinsäurefragmenten durchführt.

16. Verfahren zum Vergleichen des Methylierungsstatus von wenigstens zwei Proben von Nukleinsäurefragmenten, wobei man:
(i) das Verfahren nach einem der Ansprüche 2 bis 14 an jeder Probe von Nukleinsäurefragmenten durchführt und
(ii) die unter (i) erhaltenen Ergebnisse von einer Nukleinsäureprobe mit dem wenigstens einer weiteren Nukleinsäureprobe vergleicht.

17. Verfahren zur Diagnose oder Prognose einer Krankheit in Zusammenhang mit der Methylierung eines spezifischen Nukleinsäurefragments bei einem Individuum, wobei man:
(i) das Verfahren nach einem der Ansprüche 2 bis 14 an einer Probe von Nukleinsäurefragmenten aus dem Individuum durchführt;
(ii) das unter (i) erhaltene Ergebnis mit dem Krankheitszustand des Individuums korreliert.

18. Verfahren zum Nachweis mit der Zeit erfolgender Veränderungen der Nukleinsäuremethylierung bei einem Patienten, wobei man:
(i) eine dem Patienten zu einem Zeitpunkt entnommene Gewebeprobe nimmt,
(ii) Schritt (i) für wenigstens einen weiteren Zeitpunkt wiederholt,
(iii) Nukleinsäure aus jeder Gewebeprobe extrahiert, um für jeden Zeitpunkt eine Nukleinsäureprobe bereitzustellen, und
(iv) das Verfahren nach einem der Ansprüche 2 bis 14 an jeder Nukleinsäureprobe für jeden Zeitpunkt durchführt, um zu charakterisieren, ob und/oder in welchem Ausmaß die Nukleinsäuresequenz methyliert ist.

19. Verfahren zur Korrelation von Veränderungen der Nukleinsäuremethylierung mit den klinischen Symptomen einer Krankheit, das die Schritte nach Anspruch 18 umfasst und bei dem man ferner:
(a) bei dem Patienten beobachtete klinische Symptome einer Krankheit zu jedem Zeitpunkt aufzeichnet und
(b) die an jedem Zeitpunkt aufgezeichneten klinischen Symptome mit den unter Schritt (iv) erhaltenen Ergebnissen vergleicht.

20. Verfahren nach Anspruch 18 oder Anspruch 19, bei dem man ferner jede Gewebeprobe lagert und Schritt (ii) für mehrere Proben gleichzeitig durchführt.

21. Verfahren nach einem der Ansprüche 17 bis Anspruch 20, wobei es sich bei der Krankheit um Krebs handelt.

## Revendications

1. Procédé d'enrichissement de fragments d'acide nucléique méthylé dans un échantillon de fragments d'acide nucléique, comprenant les étapes consistant à :
(a) mettre en contact l'échantillon de fragments d'acide nucléique avec un anticorps spécifique d'un nucléoside méthylé, dans des conditions appropriées pour une liaison de l'anticorps au nucléoside méthylé ;
(b) sélectionner des fragments d'acide nucléique liés à l'anticorps spécifique du nucléoside méthylé,
**caractérisé en ce que** :
avant l'étape (a) : les brins des fragments d'acide nucléique en double brin dans l'échantillon sont séparés.

2. Procédé, selon la revendication 1, comprenant en outre l'étape (c) supplémentaire caractérisant un ou plusieurs des fragments d'acide nucléique méthylé.

3. Utilisation du procédé, selon l'une quelconque des revendications précédentes, pour détecter des allèles méthylés, de manière différentielle, dans un échantillon de fragments d'acide nucléique.

4. Procédé selon l'une quelconque des revendications précédentes, dans lequel la proportion de fragments d'acide nucléique méthylé dans l'échantillon d'acide nucléique ou prélevés à partir de celui-ci est accrue d'au moins 5 fois, d'au moins 10 fois, d'au moins 20 fois, d'au moins 30 fois, d'au moins 50 fois ou d'au moins 100 fois entre les étapes (a) et (b).

5. Procédé selon l'une quelconque des revendications précédentes, dans lequel les fragments d'acide nucléique sont des fragments d'ADN.

6. Procédé selon l'une quelconque des revendications précédentes, dans lequel le nucléoside méthylé est la méthylcytidine.

7. Procédé selon l'une quelconque des revendications précédentes, dans lequel le nucléoside méthylé est la 5-méthylcytidine.

8. Procédé selon l'une quelconque des revendications précédentes, dans lequel la sélection de fragments d'acide nucléique liés à l'anticorps est effectuée en fixant ou en liant les anticorps à un substrat solide et en séparant ce substrat solide de la phase liquide de l'échantillon.

9. Procédé selon la revendication 8, dans lequel le substrat solide se lie spécifiquement à l'anticorps qui est spécifique d'un nucléoside méthylé.

10. Procédé selon la revendication 9, dans lequel le substrat solide comprend un deuxième anticorps étant spécifique de l'anticorps spécifique du nucléoside méthylé.

11. Procédé selon l'une quelconque des revendications 8 à 10, dans lequel le substrat solide se trouve sous forme de billes.

12. Procédé selon l'une quelconque des revendications 8 à 11, dans lequel le substrat solide est magnétique.

13. Procédé, selon l'une quelconque des revendications précédentes, comprenant en outre l'étape consistant à détacher les fragments d'acide nucléique méthylé à partir de l'anticorps spécifique du nucléoside méthylé.

14. Procédé selon la revendication 13, dans lequel le détachement des fragments d'acide nucléique méthylé à partir de l'anticorps spécifique du nucléoside méthylé comprend l'incubation des fragments d'acide nucléique liés à l'anticorps avec une protéinase.

15. Procédé de caractérisation de l'état de méthylation d'un échantillon d'ADN génomique, comprenant les étapes consistant à :
(i) fragmenter l'échantillon d'ADN génomique pour donner un échantillon de fragments d'acide nucléique et
(ii) exécuter le procédé, selon l'une quelconque des revendications 2 à 14, sur l'échantillon de fragments d'acide nucléique obtenu au paragraphe (i).

16. Procédé de comparaison de l'état de méthylation d'au moins deux échantillons de fragments d'acide nucléique, comprenant les étapes consistant à :
(i) exécuter le procédé, selon l'une quelconque des revendications 2 à 14, sur chaque échantillon de fragments d'acide nucléique et
(ii) comparer les résultats obtenus au paragraphe (i), à partir d'un échantillon d'acide nucléique, avec ceux d'au moins un autre échantillon d'acide nucléique.

17. Procédé de diagnostic ou pronostic d'une maladie associée à une méthylation d'un fragment spécifique d'acide nucléique chez un individu, comprenant les étapes consistant à :
(i) exécuter le procédé, selon l'une quelconque des revendications 2 à 14, sur un échantillon de fragments d'acide nucléique provenant de l'individu ;
(ii) établir une corrélation du résultat obtenu au paragraphe (i) avec l'état de la maladie chez l'individu.

18. Procédé de détection de changements dans la méthylation de l'acide nucléique chez un patient en fonction du temps, comprenant les étapes consistant à :
(i) prélever un spécimen de tissu obtenu du patient à un temps de mesure donné ;
(ii) répéter l'étape (i) à au moins un temps de mesure supplémentaire ;
(iii) extraire l'acide nucléique de chaque spécimen de tissu pour fournir un échantillon d'acide nucléique pour chaque temps de mesure et
(iv) exécuter le procédé, selon l'une quelconque des revendications 2 à 14, sur chaque échantillon d'acide nucléique, pour chaque temps de mesure, afin de caractériser si la séquence d'acide nucléique est méthylée et/ou dans quelle mesure elle l'est.

19. Procédé de corrélation des changements de la méthylation d'un acide nucléique avec les symptômes cliniques d'une maladie, comprenant les étapes de la revendication 18 et comprenant en outre les étapes consistant à :
(a) enregistrer les symptômes cliniques d'une maladie observés chez le patient, pour chaque temps de mesure, et
(b) comparer les symptômes cliniques enregistrés à chaque temps de mesure avec les résultats obtenus à l'étape (iv).

20. Procédé, selon la revendication 18 ou la revendication 19, comprenant en outre le stockage de chaque spécimen de tissu et l'exécution de l'étape (ii) pour une pluralité de spécimens en même temps.

21. Procédé selon l'une quelconque des revendications 17 à 20, dans lequel la maladie est le cancer.
